# FASCICULE DE BREVET EUROPEEN

(11) **EP 1 252 913 B1**
(45) Date de publication et mention de la délivrance du brevet: **26.07.2006**
(21) Numéro de dépôt: 02291019.4
(22) Date de dépôt: 23.04.2002
(51) Int. Cl.: A61N 1/372, G06F 19/00

(54) **Procédé de gestion de données de protocoles cliniques portant sur l'utilisation de dispositifs médicaux implantables actifs**
Verfahren zur Verwaltung von klinischen Protokolldaten zur Verwendung in aktiven implantierbaren medizinischen Geräten
Method for managing clinical protocol data for utilisation in active implantable medical devices

(30) Priorité: 23.04.2001 FR 0105439
(43) Date de publication de la demande: 30.10.2002
(73) Titulaire: ELA MEDICAL, 92541 Montrouge (FR)
(72) Inventeur: Limousin, Marcel, 75014 Paris (FR); Nitzsche, Rémi, 78113 Bourdonne (FR)
(74) Mandataire: Dupuis-Latour, Dominique

(56) Documents cités:
- EP-A- 0 761 255
- WO-A-93/08872
- US-A- 5 660 183
- US-A- 5 693 076
- US-A- 5 724 985

## Description

L'invention concerne les "dispositifs médicaux implantables actifs" tels que définis par la Directive 90/385/CE du 20 juin 1990 du Conseil des communautés européennes. Cette définition inclut les stimulateurs cardiaques, défibrillateurs, cardioverteurs et/ou dispositifs multisite, mais aussi les appareils neurologiques, les pompes de diffusion de substances médicales, les implants cochléaires, les capteurs biologiques implantés, etc., ainsi que les dispositifs de mesure de pH ou encore de mesure d'impédance intracorporelle.

Ces dispositifs, par la suite dénommés simplement "implants", comportent une mémoire de données qui peut être lue au moyen d'un programmateur externe par des techniques de télémétrie en elles-mêmes bien connues. Le programmateur est associé à un micro-ordinateur à la disposition du praticien, comportant un écran d'affichage, un clavier pour l'entrée de commandes et la saisie de données, ainsi que divers moyens de mémorisation et de traitement de données.

Ces appareils, notamment ceux qui ont été développés récemment, peuvent faire l'objet de procédures appelées "essais cliniques" ou "protocoles cliniques", selon lesquelles, parmi la population de patients portant un modèle d'implant donné, certains patients sont sélectionnés pour faire l'objet d'un suivi plus spécifique, impliquant le plus souvent des examens complémentaires, dont les résultats seront recueillis et compilés pour étude statistique sur la population de patients sélectionnée.

L'invention propose un procédé de gestion des données de tels protocoles cliniques, permettant notamment un suivi plus régulier et exhaustif et évitant, par dématérialisation des informations recueillies, le recours à des questionnaires papier ou fiches de suivi remplies à la main par le praticien lorsqu'il examine l'un des patients concerné par le protocole clinique.

L'invention s'applique également, *mutatis mutandis,* à la gestion des données d'un "registre" ou d'une "enquête", c'est-à-dire d'une procédure impliquant (à la différence d'un protocole clinique) la totalité des patients porteurs d'un implant d'un modèle donné. Les données concernées sont des données simplement recueillies, notamment à des fins d'études épidémiologiques, lors des interrogations de l'implant, qu'il s'agisse d'interrogations de routine ou à la suite d'une évolution de la pathologie du patient, mais sans examen particulier ni schéma de suivi plus contraignant (à la différence des protocoles cliniques).

A cet effet, l'invention propose un procédé de gestion de données de protocole clinique, ou de données de registre, comprenant les étapes énoncées dans la revendication 1.

Les sous-revendications visent diverses formes de mise en oeuvre subsidiaires avantageuses.

D'autres avantages et caractéristiques de l'invention apparaîtront à la lecture de la description ci-dessous de divers aspects de la mise en oeuvre de l'invention.

L'invention part du constat qu'il est toujours difficile pour un praticien (investigateur ou médecin) de participer correctement à un protocole clinique ou à un "registre". Essentiellement, comme tout suivi d'un patient appareillé d'un implant débute par l'interrogation par un programmateur, l'invention propose d'intégrer ce programmateur à la saisie des questionnaires de protocole clinique ou de registre.

Plus précisément, la gestion du questionnaire de protocole clinique ou de registre est opérée en fin d'examen, après que le praticien ait exécuté les opérations classiques de suivi.

Ainsi, par exemple au moment où le praticien sort du programme de suivi, le programmateur lui rappelle que l'implant participe à un protocole clinique ou à une enquête.

Pour ce faire, le programmateur interroge au préalable une information spécifique de l'implant permettant de reconnaître cette participation.

Dans le cas d'un protocole clinique, il est ainsi possible de réserver dans la mémoire du stimulateur une zone mémoire où aura été préalablement inscrit le nom de code du protocole, après que le patient ait signé son consentement à participer à l'étude. Dans le cas d'un registre, il suffit au programmateur de contrôler le numéro d'identification du modèle par interrogation de l'implant pour déterminer si celui-ci est ou non concerné par un registre.

Un critère supplémentaire pour décider s'il y a lieu ou non de présenter un questionnaire au médecin peut être dérivé de la date de d'implantation de l'appareil et/ou du délai de suivi, calculé à partir de la date du jour en fonction de la date du dernier examen, mémorisée dans l'implant.

Le programmateur détermine ainsi si l'implant participe ou non à un protocole clinique ou à un registre.

Dans l'affirmative, le programmateur propose alors un questionnaire au praticien par affichage sur un écran.

En réponse, le praticien saisit les données demandées, le cas échéant après avoir procédé à des examens complémentaires (dans le cas d'un protocole clinique).

Les réponses au questionnaire sont mémorisées par le programmateur dans la base de données de ce dernier en même temps que les fichiers d'interrogation, c'est-à-dire les fichiers contenant les données de paramétrage et/ou les données Holter lues dans la mémoire de l'implant.

Une fois le questionnaire ainsi saisi et mémorisé, le programmateur peut proposer au praticien d'effectuer une interrogation complète des mémoires si cela n'a pas été fait, et/ou lui rappeler qu'il doit effectuer certains autres examens dans le cadre du protocole (épreuve d'effort, échocardiographie, etc.) et/ou lui indiquer la prochaine étape du protocole à intervenir (par exemple "revoir le patient dans six mois"), ou la date du prochain examen de routine à effectuer.

Les données ainsi recueillies et mémorisées par le programmateur sont ensuite centralisées régulièrement par disquette ou par télétransmission dans une base de données commune pour traitement statistique, sauvegarde du registre pour l'ensemble des appareils examinés, etc.

Très avantageusement, le contenu du questionnaire (la formulation des questions) est conservé dans la mémoire de l'implant, rendant ainsi la procédure de l'invention totalement indépendante du programmateur utilisé, dédié ou non, à jour ou non.

Cette manière de procéder selon l'invention présente un certain nombre d'avantages incontestables :
- elle permet d'avoir un questionnaire à la fois complet et ordonné ;
- elle permet de s'affranchir des étapes de transcription sur papier, éliminant donc les risques d'erreur et évitant le recours, comme aujourd'hui, à une double saisie des formulaires manuscrits (saisie + vérification) ;
- elle évite tout risque que le médecin ne se rappelle plus que son patient participe à une étude clinique, puisque le questionnaire est systématiquement et automatiquement présenté au praticien en fin d'examen, si (et seulement si) l'implant participe au protocole clinique ;
- le questionnaire, notamment s'il est mémorisé dans l'implant, est immédiatement mis à disposition du praticien, de façon entièrement dématérialisée sans qu'il soit nécessaire de recourir à un dossier papier.

## Revendications

1. Un procédé de gestion de données de protocole clinique, ou de données de registre, portant sur l'utilisation d'implants de type dispositifs médicaux implantables actifs, notamment stimulateurs cardiaques, défibrillateurs, cardioverteurs et/ou dispositifs multisite,
procédé respectivement **caractérisé**, dans le cas de la gestion de données de protocole clinique, par les étapes suivantes :
1) initialisation préalable des implants participant à un protocole donné, par inscription dans une zone d'identification de protocole prévue dans la mémoire de l'implant d'un code de protocole commun à tous lesdits implants participant au protocole donné,
2) à chaque interrogation d'un quelconque implant au moyen d'un programmateur :
a) recherche d'une zone d'identification de protocole dans la mémoire de l'implant et lecture du contenu de celle-ci,
b) contrôle de présence d'un code de protocole dans cette zone,
c) en cas de présence d'un code de protocole :
c1) reconnaissance du code de protocole par le programmateur,
c2) présentation, par affichage sur un écran du programmateur, d'un questionnaire propre au protocole identifié par ledit code,
c3) saisie de réponses audit questionnaire au moyen du programmateur,
c4) mémorisation de ces réponses dans une mémoire du programmateur.
et, dans le cas de la gestion de données de registre, par les étapes suivantes, exécutées à chaque interrogation d'un implant au moyen d'un programmateur :
a) lecture dans la mémoire de l'implant du contenu de la zone d'identification du modèle d'implant,
b) reconnaissance du modèle d'implant par le programmateur,
c) présentation, par affichage sur un écran du programmateur, d'un questionnaire propre à ce modèle d'implant,
d) saisie de réponses audit questionnaire au moyen du programmateur,
e) mémorisation de ces réponses dans une mémoire du programmateur.

2. Le procédé de la revendication 1, comprenant en outre des étapes de :
- lecture par le programmateur de données de paramétrage et/ou de données Holter contenues dans une mémoire de l'implant, et
- mémorisation de ces données dans la mémoire du programmateur conjointement avec les réponses au questionnaire
ces étapes étant, dans le cas de la gestion de données de protocole clinique, exécutées en cas de présence d'un code de protocole.

3. Le procédé de l'une des revendications 1 ou 2, dans lequel il est en outre prévu périodiquement une étape de :
- collecte et centralisation pour traitement ultérieur des réponses, ainsi que le cas échéant des données de paramétrage et/ou des données Hotter, relatives à une pluralité d'implants et mémorisées dans un programmateur ou une pluralité de programmateurs.

4. Le procédé de la revendication 1, comprenant en outre une étape de :
- présentation, par affichage sur l'écran du programmateur, d'un message de rappel d'examens complémentaires à effectuer et/ou d'un message informatif de la prochaine étape de protocole à intervenir.

5. Le procédé de la revendication 1, comprenant également dans le cas de la gestion de données de protocole clinique à l'étape a) la lecture dans la mémoire de l'implant de la date d'implantation et/ou de la date de la dernière interrogation de l'implant, la présentation du questionnaire à l'étape c) étant conditionnée par au moins l'une de ces dates.

6. Le procédé de la revendication 1, dans lequel ledit questionnaire est mémorisé dans la mémoire de l'implant et lu dans cette dernière pour être présenté à l'étape c).

## Claims

1. A process for managing clinical protocol data or registry data, having as a subject the use of implants of an active implantable medical device type, such as pacemakers, defibrillators, cardioverters and/or multisite devices, a process respectively **characterised, in** the case of managing clinical protocol data, by the following steps:
1) preliminary initialisation of implants taking part in a given protocol by writing a protocol code common to all implants taking part in the given protocol in a protocol identification zone provided for in the implant's memory,
2) at each interrogation of any implant using a programming device:
a) search for a protocol identification zone in the memory of the implant and reading the contents thereof,
b) check of the presence of a protocol code in this zone,
c) in the event of the presence of a protocol code:
c1) recognition of the protocol code by the programming device,
c2) presentation of a questionnaire inherent to the protocol identified by said code by display on a screen of the programming device,
c3) capture of answers to said questionnaire using the programming device,
c4) storage of these answers in a memory of the programming device;
and, in the case of managing register data, by the following steps, which are executed at each interrogation of an implant using the programming device:
a) reading in the implant's memory the contents of the identification zone of the implant model,
b) recognition of the implant's model by the programming device,
c) presentation, by display on a screen of the programming device, of a questionnaire inherent to this implant model,
d) capture of answers to said questionnaire using the programming device,
e) storage of these answers in a memory of the programming device.

2. The process of claim 1, further comprising steps of:
- reading, by the programming device, of parametric data and/or Holter data contained in a memory of the implant, and
- storage of this data in the memory of the programming device jointly with the answers to the questionnaire,
these steps being executed, in the case of managing clinical protocol data, in the case of the presence of a protocol code.

3. The process of claim 1 or 2, wherein there is further periodically provided a step of:
- collection and centralisation, for later processing, of the answers, as well as, if applicable, of the parametric data and/or the Holter data relative to a plurality of implants and stored in a programming device or a plurality of programming devices.

4. The process of claim 1, further comprising a step of:
- presentation, by display on the programming device's screen, of a reminder message of complementary examinations to carry out and/or an informative message about the next stage of the protocol that is to take place.

5. The process of claim 1, equally comprising, in the case of managing clinical protocol data, at step a), the reading in the implant's memory of the implantation date and/or the date of the last interrogation of the implant, wherein the presentation of the questionnaire at step c) is conditioned by at least one of these dates.

6. The process of claim 1, wherein said questionnaire is stored in the implant's memory and read in the latter to be presented at step c).

## Patentansprüche

1. Verfahren zur Verwaltung von Daten eines klinischen Protokolls oder Registerdaten, betreffend die Verwendung von Implantaten vom Typ aktiver implantierbarer medizinischer Vorrichtungen, insbesondere Herzschrittmacher, Defibrillatoren, Kardioverter und/oder "Mulrisite"-Vorrichtungen, ein Verfahren, dass jeweils im Falle der Verwaltung von Daten eines klinischen Protokolls durch die folgenden Schritte **gekennzeichnet** ist:
1) Vorabinitialisierung der an einem gegebenen Protokoll teilnehmenden Implantate durch Eintragung eines Protokollcodes, der allen an dem gegebenen Protokoll teilnehmenden Implantaten gemein ist, in eine Zone zur Protokollidentifikation, die in dem Speicher des Implantats vorgesehen ist,
2) bei jeder Befragung irgendeines Implantats mittels einer Programmiereinrichtung:
a) Suchen einer Zone zur Protokollidentifikation im Speicher des Implantats und Auslesen ihres Inhalts,
b) Überprüfung der Anwesenheit eines Protokollcodes in dieser Zone,
c) im Falle der Anwesenheit eines Protokollcodes:
c1) Erkennung des Protokollcodes durch die Programmiereinrichtung,
c2) Präsentation eines dem durch den Code identifizierten Protokoll eigenen Fragebogens durch Anzeige auf einem Bildschirm der Programmiereinrichtung,
c3) Erfassung von Antworten auf den Fragebogen mittels der Programmiereinrichtung,
c4) Speicherung dieser Antworten in einem Speicher der Programmiereinrichtung;
und, im Falle der Verwaltung von Registerdaten, durch die folgenden Schritte, die bei jeder Befragung eines Implantats mittels der Programmiereinrichtung durchgeführt werden:
a) Auslesen des Inhalts der Identifikationszone des Implantatmodels aus dem Speicher des Implantats,
b) Erkennung des Implantatmodels durch die Programmiereinrichtung,
c) Präsentation eines diesem Implantatmodel eigenen Fragebogens durch Anzeige auf einem Bildschirm der Programmiereinrichtung,
d) Erfassung von Antworten auf den Fragebogen mittels der Programmiereinrichtung,
e) Speicherung dieser Antworten in einem Speicher der Programmiereinrichtung.

2. Verfahren nach Anspruch 1, weiterhin mit Schritten:
- zum Auslesen von Parameterdaten und/oder Holterdaten durch die Programmiereinrichtung, die in einem Speicher des Implantats enthalten sind, und
- Speicherung dieser Daten im Speicher der Programmiereinrichtung zusammen mit den Antworten auf den Fragebogen,
wobei diese Schritte im Falle der Verwaltung von Daten eines klinischen Protokolls im Falle der Anwesenheit eines Protokollcodes durchgeführt werden.

3. Verfahren nach Anspruch 1 oder 2, bei welchem weiterhin periodisch ein Schritt zur:
- Sammlung und Zentralisierung für die spätere Verarbeitung der Antworten sowie gegebenenfalls der Parameterdaten und/oder Holterdaten bezüglich einer Mehrzahl Implantate, die in einer Programmiereinrichtung oder einer Mehrzahl Programxniereinrichtungen gespeichert sind,
vorgesehen ist.

4. Verfahren nach Anspruch 1, weiterhin mit einem Schritt zur:
- Präsentation einer Nachricht zur Erinnerung an zusätzliche vorzunehmende Untersuchungen und/oder einer Informationsnachricht über den nächsten anstehenden Protokollschritt durch Anzeige auf dem Bildschirm der Programmiereinrichtung.

5. Verfahren nach Anspruch 1, welches ebenfalls im Falle der Verwaltung von Daten eines klinischen Protokolls beim Schritt a) das Auslesen des Implantierungsdatums und/oder des Datums der letzten Abfragung des Implantats aus dem Speicher des Implantats umfasst, wobei die Präsentation des Fragebogens beim Schritt c) durch wenigstens eine dieser Daten konditioniert ist.

6. Verfahren nach Anspruch 1, bei welchem der Fragebogen im Speicher des Implantats gespeichert ist und aus letzterem ausgelesen wird, um beim Schritt c) präsentiert zu werden.
